(19) **Europäisches Patentamt**
**European Patent Office**
**Office européen des brevets**

(11) **EP 2 145 899 A1**

(12) **EUROPEAN PATENT APPLICATION**

(43) Date of publication:
**20.01.2010 Bulletin 2010/03**

(51) Int Cl.:
*C07K 16/10* (2006.01)   *G01N 33/569* (2006.01)

(21) Application number: **09165635.5**

(22) Date of filing: **16.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **18.07.2008 JP 2008187368**

(71) Applicant: **Sysmex Corporation**
**Kobe-shi, Hyogo 651-0073 (JP)**

(72) Inventors:
• **Asaeda, Ayumi**
  **Hyogo 651-0073 (JP)**

• **Okitsu, Naoya**
  **Hyogo 651-0073 (JP)**
• **Kumamoto, Hiroshi**
  **Hyogo 651-0073 (JP)**
• **Sakaguchi, Kouji**
  **Hyogo 651-0073 (JP)**
• **Hasegawa, Takehiro**
  **Hyogo 651-0073 (JP)**

(74) Representative: **Paemen, Liesbet R.J. et al**
**De Clercq, Brants & Partners**
**E. Gevaertdreef 10 A**
**9830 Sint-Martens-Latem (BE)**

(54) **RS virus detecting kit using anti-RS virus monoclonal antibody, immuno-chromatographic test device, and new anti-RS virus monoclonal antibody**

(57)    A kit or an immuno-chromatographic test device for detection of respiratory syncytial virus (RSV), comprising at least an RSV F protein-recognizing anti-RSV monoclonal antibody produced by hybridoma RSF2-412. An anti-RSV monoclonal antibody recognizing an RS virus F protein, which is selected from the group consisting of an antibody produced by hybridoma RSF2-412, an antibody produced by hybridoma RSF1-1565, and an antibody produced by hybridoma RSF6-255.

SEQUENCE LISTING

<110> Sysmex Corporation

<120> Kit and immunochromatography test device for RSV detection using anti-RSV monoclonal antibody

<130> 08-007JP

<160> 8

<170> PatentIn version 3.1

<210> 1
<211> 574
<212> PRT
<213> respiratory syncytial virus

<400> 1

Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Ala Asn Asn Arg Ala Arg Arg Glu Leu Pro
            100                 105                 110

Arg Phe Met Asn Tyr Thr Leu Asn Asn Thr Lys Lys Thr Asn Val Thr
            115                 120                 125

Leu Ser Lys Lys Arg Lys Arg Arg Phe Leu Gly Phe Leu Leu Gly Val
    130                 135                 140

16

Gly Ser Ala Ile Ala Ser Gly Thr Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
    355                 360                 365

```
Thr Met Asn Ser Leu Thr Leu Pro Ser Glu Val Asn Leu Cys Asn Val
    370             375             380

Asp Ile Phe Asn Pro Lys Tyr Asp Cys Lys Ile Met Thr Ser Lys Thr
385             390             395             400

Asp Val Ser Ser Ser Val Ile Thr Ser Leu Gly Ala Ile Val Ser Cys
            405             410             415

Tyr Gly Lys Thr Lys Cys Thr Ala Ser Asn Lys Asn Arg Gly Ile Ile
            420             425             430

Lys Thr Phe Ser Asn Gly Cys Asp Tyr Ala Ser Asn Lys Gly Val Asp
        435             440             445

Thr Val Ser Val Gly Asn Thr Leu Tyr Tyr Val Asn Lys Gln Glu Gly
    450             455             460

Lys Ser Leu Tyr Val Lys Gly Glu Pro Ile Ile Asn Phe Tyr Asp Pro
465             470             475             480

Leu Val Phe Pro Ser Asp Glu Phe Asp Ala Ser Ile Ser Gln Val Asn
            485             490             495

Glu Lys Ile Asn Gln Ser Leu Ala Phe Ile Arg Lys Ser Asp Glu Leu
            500             505             510

Leu His His Val Asn Ala Gly Lys Ser Thr Thr Asn Ile Met Ile Thr
        515             520             525

Thr Ile Ile Ile Val Ile Ile Val Ile Leu Leu Ser Leu Ile Ala Val
    530             535             540

Gly Leu Leu Leu Tyr Cys Lys Ala Arg Ser Thr Pro Val Thr Leu Ser
545             550             555             560

Lys Asp Gln Leu Ser Gly Ile Asn Asn Ile Ala Phe Ser Asn
            565             570
```

```
<210>  2
<211>  574
<212>  PRT
```

<213>   respiratory syncytial virus

<400>   2

```
Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
            85                  90                  95

Met Gln Ser Thr Pro Ala Ala Asn Asn Arg Ala Arg Arg Glu Leu Pro
            100                 105                 110

Arg Phe Met Asn Tyr Thr Leu Asn Asn Thr Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Thr Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205
```

Lys Gln Ser Cys Arg Ile Ser Asn Ile Glu Thr Val Ile Glu Phe Gln
210                      215               220

Gln Lys Asn Asn Arg Leu Leu Glu Ile Thr Arg Glu Phe Ser Val Asn
225               230               235               240

Ala Gly Val Thr Thr Pro Val Ser Thr Tyr Met Leu Thr Asn Ser Glu
               245               250               255

Leu Leu Ser Leu Ile Asn Asp Met Pro Ile Thr Asn Asp Gln Lys Lys
          260               265               270

Leu Met Ser Asn Asn Val Gln Ile Val Arg Gln Gln Ser Tyr Ser Ile
     275               280               285

Met Ser Ile Ile Lys Glu Glu Val Leu Ala Tyr Val Val Gln Leu Pro
290               295               300

Leu Tyr Gly Val Ile Asp Thr Pro Cys Trp Lys Leu His Thr Ser Pro
305               310               315               320

Leu Cys Thr Thr Asn Thr Lys Glu Gly Ser Asn Ile Cys Leu Thr Arg
               325               330               335

Thr Asp Arg Gly Trp Tyr Cys Asp Asn Ala Gly Ser Val Ser Phe Phe
          340               345               350

Pro Gln Ala Glu Thr Cys Lys Val Gln Ser Asn Arg Val Phe Cys Asp
     355               360               365

Thr Met Asn Ser Leu Thr Leu Pro Ser Glu Val Asn Leu Cys Asn Val
     370               375               380

Asp Ile Phe Asn Pro Lys Tyr Asp Cys Lys Ile Met Thr Ser Lys Thr
385               390               395               400

Asp Val Ser Ser Ser Val Ile Thr Ser Leu Gly Ala Ile Val Ser Cys
               405               410               415

Tyr Gly Lys Thr Thr Cys Thr Ala Ser Asn Lys Asn Arg Gly Ile Ile
          420               425               430

```
Lys Thr Phe Ser Asn Gly Cys Asp Tyr Ala Ser Asn Lys Gly Val Asp
        435             440             445

Thr Val Ser Val Gly Asn Thr Leu Tyr Tyr Val Asn Lys Gln Glu Gly
        450             455             460

Lys Ser Leu Tyr Val Lys Gly Glu Pro Ile Ile Asn Phe Tyr Asp Pro
465             470             475             480

Leu Val Phe Pro Ser Asp Glu Phe Asp Ala Ser Ile Ser Gln Val Asn
                485             490             495

Glu Lys Ile Asn Gln Ser Leu Ala Phe Ile Arg Lys Ser Asp Glu Leu
            500             505             510

Leu His His Val Asn Ala Gly Lys Ser Thr Thr Asn Ile Met Ile Thr
        515             520             525

Thr Ile Ile Ile Val Ile Ile Val Ile Leu Leu Ser Leu Ile Ala Val
        530             535             540

Gly Leu Leu Leu Tyr Cys Lys Ala Arg Ser Thr Pro Val Thr Leu Ser
545             550             555             560

Lys Asp Gln Leu Ser Gly Ile Asn Asn Ile Ala Phe Ser Asn
                565             570
```

```
<210>   3
<211>   23
<212>   DNA
<213>   Artificial

<220>
<223>   Primer for reverse transcription

<400>   3
atggagttgc caatcctcaa agc                                   23


<210>   4
<211>   31
<212>   DNA
<213>   Artificial

<220>
<223>   Primer for PCR
```

```
<400>  4
atggatccat ggagttgcca atcctcaaag c
31


<210>  5
<211>  52
<212>  DNA
<213>  Artificial

<220>
<223>  Primer for PCR

<400>  5
tagaattcta gtgatggtga tggtgatgat ttgtggtgga tttaccagca tt
52


<210>  6
<211>  26
<212>  DNA
<213>  Artificial

<220>
<223>  Primer for cycle sequencing

<400>  6
tttcttggtt ttttgttagg tgttgg
26


<210>  7
<211>  27
<212>  DNA
<213>  Artificial

<220>
<223>  Primer for cycle sequencing

<400>  7
tttaacatta ccaagtgaaa taaatct
27


<210>  8
<211>  26
<212>  DNA
<213>  Artificial

<220>
<223>  Primer for cycle sequencing

<400>  8
tcttcttttc cttttcttgc ttaatg
26
```

SEQUENCE LISTING

<110>  Sysmex Corporation

<120>  Kit and immunochromatography test device for RSV detection
using anti-RSV monoclonal antibody

<130>  08-007JP

<160>  8

<170>  PatentIn version 3.1

<210>  1
<211>  574
<212>  PRT
<213>  respiratory syncytial virus

<400>  1

Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Ala Asn Asn Arg Ala Arg Arg Glu Leu Pro
            100                 105                 110

Arg Phe Met Asn Tyr Thr Leu Asn Asn Thr Lys Lys Thr Asn Val Thr
            115                 120                 125

Leu Ser Lys Lys Arg Lys Arg Arg Phe Leu Gly Phe Leu Leu Gly Val
        130                 135                 140

```
Gly Ser Ala Ile Ala Ser Gly Thr Ala Val Ser Lys Val Leu His Leu
145             150             155                 160

Glu Gly Glu Val Asn Lys Ile Lys Ser Ala Leu Leu Ser Thr Asn Lys
                165             170                 175

Ala Val Val Ser Leu Ser Asn Gly Val Ser Val Leu Thr Ser Lys Val
            180             185                 190

Leu Asp Leu Lys Asn Tyr Ile Asp Lys Gln Leu Leu Pro Ile Val Asn
        195             200                 205

Lys Gln Ser Cys Arg Ile Ser Asn Ile Glu Thr Val Ile Glu Phe Gln
    210             215                 220

Gln Lys Asn Asn Arg Leu Leu Glu Ile Thr Arg Glu Phe Ser Val Asn
225             230             235                 240

Ala Gly Val Thr Thr Pro Val Ser Thr Tyr Met Leu Thr Asn Ser Glu
            245             250                 255

Leu Leu Ser Leu Ile Asn Asp Met Pro Ile Thr Asn Asp Gln Lys Lys
            260             265                 270

Leu Met Ser Asn Asn Val Gln Ile Val Arg Gln Gln Ser Tyr Ser Ile
    275             280                 285

Met Ser Ile Ile Lys Glu Glu Val Leu Ala Tyr Val Val Gln Leu Pro
    290             295                 300

Leu Tyr Gly Val Ile Asp Thr Pro Cys Trp Lys Leu His Thr Ser Pro
305             310             315                 320

Leu Cys Thr Thr Asn Thr Lys Glu Gly Ser Asn Ile Cys Leu Thr Arg
            325             330                 335

Thr Asp Arg Gly Trp Tyr Cys Asp Asn Ala Gly Ser Val Ser Phe Phe
        340             345                 350

Pro Gln Ala Glu Thr Cys Lys Val Gln Ser Asn Arg Val Phe Cys Asp
    355             360                 365
```

```
Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Ala Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His His Val Asn Ala Gly Lys Ser Thr Thr Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570
```

```
<210>  2
<211>  574
<212>  PRT
```

<213> respiratory syncytial virus

<400> 2

Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
                20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Ala Asn Asn Arg Ala Arg Arg Glu Leu Pro
                100                 105                 110

Arg Phe Met Asn Tyr Thr Leu Asn Asn Thr Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Thr Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
                180                 185                 190

Leu Asp Leu Lys Asn Tyr Ile Asp Lys Gln Leu Leu Pro Ile Val Asn
        195                 200                 205

```
Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
                260                 265                 270

Leu Met Ser Asn Asn Val Gln Ile Val Arg Gln Gln Ser Tyr Ser Ile
                275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
                340                 345                 350

Pro Gln Ala Glu Thr Cys Lys Val Gln Ser Asn Arg Val Phe Cys Asp
    355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Thr Cys Thr Ala Ser Asn Lys Asn Arg Gly Ile Ile
                420                 425                 430
```

```
Lys Thr Phe Ser Asn Gly Cys Asp Tyr Ala Ser Asn Lys Gly Val Asp
    435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
                500                 505                 510

Leu His His Val Asn Ala Gly Lys Ser Thr Thr Asn Ile Met Ile Thr
    515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570
```

```
<210>  3
<211>  23
<212>  DNA
<213>  Artificial

<220>
<223>  Primer for reverse transcription

<400>  3
atggagttgc caatcctcaa agc                                        23


<210>  4
<211>  31
<212>  DNA
<213>  Artificial

<220>
<223>  Primer for PCR
```

```
<400>  4
atggatccat ggagttgcca atcctcaaag c
31


<210>  5
<211>  52
<212>  DNA
<213>  Artificial

<220>
<223>  Primer for PCR

<400>  5
tagaattcta gtgatggtga tggtgatgat ttgtggtgga tttaccagca tt
52


<210>  6
<211>  26
<212>  DNA
<213>  Artificial

<220>
<223>  Primer for cycle sequencing

<400>  6
tttcttggtt ttttgttagg tgttgg
26


<210>  7
<211>  27
<212>  DNA
<213>  Artificial

<220>
<223>  Primer for cycle sequencing

<400>  7
tttaacatta ccaagtgaaa taaatct
27


<210>  8
<211>  26
<212>  DNA
<213>  Artificial

<220>
<223>  Primer for cycle sequencing

<400>  8
tcttcttttc cttttcttgc ttaatg
26
```

**Description**

FIELD OF THE INVENTION

The present invention relates to a respiratory syncytial virus detecting kit using anti-respiratory syncytial virus monoclonal antibody which can detect respiratory syncytial virus with a high level of sensitivity, and an immuno-chromatographic test device. The present invention also relates to an anti- respiratory syncytial virus monoclonal antibody which can be used for manufacturing the kit or the device.

BACKGROUND

Respiratory syncytial virus (hereinafter referred to as "RSV" or "RS virus") is a main causal virus developing respiratory infections in infants. Usually, infants infected with RSV develop cold-like slight symptoms. However, there are cases where immunocompromised infants or elderly persons when infected with RS virus develop bronchiolitis and even die of breathing difficulties. Accordingly, it has been desired to easily and rapidly detect infection with this virus.
RSV is classified into subtypes (types A and B) based mainly on serological differences. A Long strain, A2 strain or the like is known as type-A RSV. A 9320 strain, B1 wild type strain or the like is known as type-B RSV.
In an envelope of RSV, there are a glycoprotein G protein and a cell fusion-related F protein. It is known that the amino acid sequence of G protein is considerably different between the subtypes (types A and B) of RSV. On the other hand, it is known that the amino acid sequence of F protein is slightly different even between the subtypes (types A and B) of RSV.
Some anti-RSV monoclonal antibodies recognizing RSV are known. WO 98/19704 discloses modified monoclonal antibodies binding to the F protein of RSV and having a neutralizing activity. WO 03/063767 discloses human RSV antibodies. WO 96/40252 also discloses human monoclonal antibodies binding to the F protein of RSV.
Anti-RSV monoclonal antibodies binding to the F protein of RSV, which are obtained from various clones, are commercially available.
Further, RSV detection kits based on immuno-chromatographic techniques using anti-RSV monoclonal antibodies, for example BD RSV Examan (Becton Dickinson), Check RSV (Alfresa-Pharma Co. Japan), BinaxNOW (Eiken Chemical Co., Ltd.), Immunocard (TFB, Inc.), and Poctem S RSV (Sysmex Corporation), are also commercially available.
However, these conventional kits for detection of RSV, when used to detect RSV, are poor in sensitivity and sometimes judge 30% to 40% of positive samples to be negative.

SUMMARY

The scope of the present invention is defined solely by the appended claims, and is not affected to any degree by the statements within this summary.
A first aspect of the present invention is a kit for detection of RSV, comprising an RS virus F protein-recognizing anti-RSV monoclonal antibody produced by hybridoma RSF2-412 deposited on April 20, 2009, under Accession No. NITE BP-601, with Incorporated Administrative Agency National Institute of Technology and Evaluation Patent Microorganisms Depositary.
A second aspect of the present invention is an immuno-chromatographic test device for detection of RSV, comprising at least an RSV F protein-recognizing anti-RSV monoclonal antibody produced by hybridoma RSF2-412.
A third aspect of the present invention is an anti-RSV monoclonal antibody recognizing an RS virus F protein, which is selected from the group consisting of an antibody produced by hybridoma RSF2-412, an antibody produced by hybridoma RSFI-1565, and an antibody produced by hybridoma RSF6-255.

BRIEF DESCRIPTION OF THE DRAWINGS

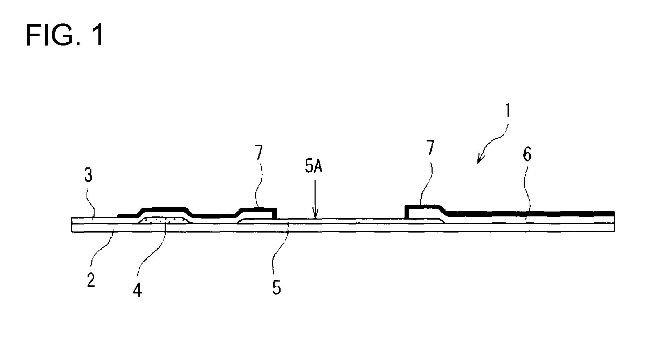

FIG. 1 shows an embodiment of the immuno-chromatographic test device of the present invention.

DETAILED DESCRIPTION OF THE PREFERRED EMBODIMENTS

The preferred embodiments of the present invention are described hereinafter with reference to the drawings.

(Hybridoma and anti-RSV monoclonal antibody)

The anti-RSV monoclonal antibody used in the RSV detection kit and immuno-chromatographic test device of the present invention is produced by hybridoma RSF2-412 (referred to hereinafter as "hybridoma 412"). This antibody is referred to hereinafter as "antibody 412".

The antibody of the present invention can be obtained from a hybridoma created by a method known per se. That is, an RSV antigen, preferably the RSV F protein, which is optionally mixed with a suitable adjuvant, is used to immunize a suitable mammal (for example, a mouse, a rat or the like). Then, antibody-producing cells such as spleen cells, lymph node cells or B lymphocytes from the animal can be fused with myeloma cells derived from a suitable mammal (for example, a mouse, a rat or the like) to yield hybridoma. Usually, the antibody-producing cells and the myeloma cells are derived from the same kind of animals.

Cell fusion can be carried out, for example, by the PEG method wherein antibody-producing cells and myeloma cells are fused with each other in the presence of polyethylene glycol (PEG) or the like in a suitable medium. After cell fusion, the resulting hybridoma is selected in a selective medium such as HAT medium and screened by a conventional method (for example, enzyme immunoassay (EIA)) for their ability to produce antibodies recognizing the RSV F protein. Then, a hybridoma producing an appropriate antibody is cloned by a conventional method (for example, limiting dilution method), thereby selecting the hybridoma producing a monoclonal antibody.

In the present invention, hybridoma 412 was obtained by fusing mouse spleen cells obtained by immunizing a mouse with the RSV F protein, with mouse myeloma cells, as shown later in the Examples. The hybridoma 412 was deposited on June 26, 2008, with Incorporated Administrative Agency National Institute of Technology and Evaluation Patent Microorganisms Depositary (2-5-8 Kazusakamatari Kisarazu-shi, Chiba-ken, Japan) and converted to international deposition (Date of conversion of deposit, April 20, 2009; Accession No. NITE BP-601).

In the present invention, hybridoma RSF1-1565 (hereinafter referred to as "hybridoma 1565") and hybridoma RSF6-255 (hereinafter referred to as "hybridoma 255") were obtained according to the same method as described above. The hybridoma 1565 was deposited on July 19, 2007, with International Patent Organism Depositary National Institute of Advanced Industrial Science and Technology (AIST) (Tsukuba Central 6, 1-1, Higashi 1-chome Tsukuba-shi, Ibaraki-ken, Japan) and converted to international deposition (Date of conversion of deposit, April 20, 2009; Accession No. FERM BP-11119). The hybridoma 255 was deposited on June 26, 2008, with International Patent Organism Depositary National Institute of Advanced Industrial Science and Technology (AIST) and converted to international deposition (Date of conversion of deposit, April 20, 2009; Accession No. NITE BP-602). Hereinafter, the anti-RSV monoclonal antibodies produced by the hybridomas 1565 and 255 are referred to as antibodies 1565 and 255, respectively.

A specific method of obtaining the hybridomas 412, 1565 and 255 will be described later in the Examples.

The antibodies 412, 1565 and 255 produced by the respective hybridomas can bind to the F protein of RSV The amino acid sequence of F protein of RSV Long strain is known and set forth in SEQ ID NO: 1.

As will be described later in the Examples, these 3 antibodies are considered to recognize different epitopes of the RSV F protein.

In this specification, the "antibody" includes an antibody and an antibody fragment and modified antibody having an RSV F protein-binding property equivalent to that of the antibody. The antibody fragment includes Fab fragment, F(ab')$_2$ fragment, Fab' fragment and sFv fragment.

(Kit for detection of RSV)

The kit for detection of RSV in the present invention contains at least antibody 412 produced by hybridoma 412.

In this specification, the "kit for detection of RSV" contains a plurality of constituent elements serving as a group wherein the plurality of constituent elements can be used in combination to detect RSV. The kit for detection of RSV in the present invention consists of a first constituent element containing the antibody 412 and other constituent elements. These constituent elements can be a solution which does or does not contain the antibody, a solid carrier which does or does not contain the antibody, or a combination thereof.

Preferably, the kit of the present invention further contains an anti-RSV antibody (hereinafter referred to as "other antibody") recognizing an epitope of the RSV F protein which is different from the epitope recognized by the antibody 412. The other antibody may be one or more antibodies, preferably two antibodies. More preferably, the kit of the present invention contains three antibodies, that is, the antibody 412 and two other anti-RSV antibodies, thereby attaining higher sensitivity in detection of RSV than by the conventional RSV detection kits.

Whether the epitopes recognized by the other antibodies are identical with the epitope recognized by the antibody 412 can be examined by a known method. The known method includes a competitive inhibition test. The competitive inhibition test is a method wherein the ability of an objective antibody to competitively inhibit the binding of an arbitrary antibody to an antigen is examined thereby determining whether the epitopes to which the respective antibodies bind are the same or not. More specifically, the method carried out under the conditions shown later in Example 3 can be mentioned. The arbitrary antibody is not particularly limited as long as it is an antibody other than the objective antibody.

For example, when the binding, which can be inhibited by the antibody 412, of an arbitrary antibody to an RSV antigen cannot be inhibited by a certain anti-RSV antibody in the competitive inhibition test, then this anti-RSV antibody is the "other antibody" mentioned above. When the binding, which cannot be inhibited by the antibody 412, of an arbitrary antibody to an RSV antigen can be inhibited by a certain anti-RSV antibody, then this anti-RSV antibody is also the "other antibody" mentioned above.

In the competitive inhibition test, the phrase "can be inhibited" means that the binding of an arbitrary antibody to an RSV antigen is inhibited by 30% or more, preferably 50% or more, more preferably 90% or more.

In the competitive inhibition test, the phrase "cannot be inhibited" means that the binding of an arbitrary antibody to an RSV antigen is inhibited by less than 30%, preferably less than 10%, most preferably 0%.

The antibody which can be contained in the kit of the present invention as the other antibody than the antibody 412 is preferably an anti-RSV monoclonal antibody not binding to a protein having the amino acid sequence set forth in SEQ ID NO: 2. The amino acid sequence of SEQ ID NO: 2 is the same as the amino acid sequence (SEQ ID NO: 1) of the RSV F protein except that a lysine residue at position 421 is changed by mutation to a threonine residue. The anti-RSV monoclonal antibody not binding to a protein having the amino acid sequence of SEQ ID NO: 2 includes, for example, the antibody 1565 (see Example 5).

Other preferable antibodies include the antibody 255. Both the antibodies 1565 and 255 are contained particularly preferably as the other antibodies in the kit.

The kit of the present invention is not particularly limited as long as it contains the antibody 412 and contains constituent elements enabling an immunoassay to detect an antigen through an antigen-antibody reaction. Such constituent elements are known to those killed in the art.

The immunoassay includes immunoprecipitation and labeling immunoassay. The labeling immunoassay includes immuno-chromatography, enzyme immunoassay (EIA), radioimmunoassay (RIA), fluorescent immunoassay (FIA) and chemiluminescent immunoassay.

(Labeled antibody)

The kit of the present invention is preferably a kit intended for labeling immunoassay. In such a kit, at least one of the anti-RSV monoclonal antibodies used is labeled with a labeling substance. The antibody labeled with a labeling substance is preferably an anti-RSV monoclonal antibody not binding to a protein having the amino acid represented by SEQ ID NO: 2, particularly preferably the antibody 1565.

The labeling substance is not particularly limited as long as it is a labeling substance that can be used in usual immunoassays. Examples of the labeling substance include an enzyme, a fluorescent substance, a radioisotope, an insoluble granular substance, and the like.

The enzyme includes an alkaline phosphatase, peroxidase, glucose oxidase, tyrosinase, acid phosphatase, and the like.

The fluorescent substance includes fluorescein isothiocyanate (FITC), green fluorescence protein (GFP), luciferin, and the like.

The radioisotope includes $^{125}$I, $^{14}$C, $^{32}$P, and the like.

The insoluble granular substance includes, for example, agar, agarose, crosslinked agarose, crosslinked alginic acid, crosslinked guar gum, cellulose esters such as nitrocellulose and carboxyl cellulose, gelatin, crosslinked gelatin, latex, rubber, natural or synthetic resins such as polyethylene, polypropylene, polystyrene, styrene-butadiene copolymers, polyvinyl chloride, polyvinyl acetate, polyacrylamide, polymethacrylate, styrene-methacrylate copolymers, polyglycidyl methacrylate and acrolein-ethyleneglycol dimethacrylate copolymers, and derivatives of these resins. Labeled particles obtained by labeling particles consisting of an inorganic material such as glass (for example activated glass), silica gel, kaolin, talc, silica-alumina, alumina or barium sulfate, with pigment molecules, fluorescent molecules, magnetic particles or the like can also be used as the insoluble granular substance. Further, metal colloid particles such as gold colloids, erythrocytes, and the like can also be used as the insoluble granular substance. The insoluble granular substance is preferably metal colloid particles or colored synthetic polymer particles prepared by labeling the synthetic polymer with pigment molecules.

(Antibody immobilized on a solid phase)

The labeling immunoassay is preferably a solid phase method of using the binding of an antibody to a solid phase.

In the solid phase method, at least one of the anti-RSV monoclonal antibodies used is immobilized on a solid phase.

The antibody immobilized on a solid phase is preferably at least one selected from the antibodies 412 and 255.

More preferably, these two antibodies are immobilized on a solid phase.

The solid phase used in the solid phase method includes a microtiter plate, a membrane, particles, and the like.

The material of the solid phase includes polymer materials such as latex, rubber, polyethylene, polypropylene, polystyrene, styrene-butadiene copolymers, polyvinyl chloride, polyvinyl acetate, polyacrylamide, polymethacrylate, styrene-methacrylate copolymers, polyglycidyl methacrylate, acrolein-ethyleneglycol dimethacrylate copolymers, silicone polyvinylidene difluoride (PVDF), celluloses (nitrocellulose, cellulose acetate, and the like), nylon (for example, a modified nylon into which a carboxyl group, and an amino group which may be substituted with an alkyl group, have been introduced); agarose; gelatin; erythrocytes; and inorganic materials such as silica gel, glass, inert alumina, and magnetic substance. The solid phase can be produced from one of these materials or a combination thereof.

(Sample)

The sample examined with the RSV detection kit of the present invention includes biological samples collected from patients suspected of being infected with RSV as well as samples obtained by pretreatment of the biological samples. The biological samples include blood, serum, stools, urine, saliva, nasal discharge, swabs, sweat, and tears. The pretreatment involves dissolving a biological sample in a suitable pretreatment reagent and then filtering the resulting solution.

(Constituent elements of the kit)

When the solid phase is a microtiter plate, the kit of the present invention contains a microtiter plate and a solution containing at least the antibody 412. Alternatively, the kit of the present invention may be a kit containing a microtiter plate on which the antibody 412 is immobilized.

When the solid phase is a membrane, the kit of the present invention contains a membrane and a solution containing at least the antibody 412. Alternatively, the kit of the present invention may be a kit containing a membrane on which the antibody 412 is carried or immobilized.

When the solid phase is particles, the kit of the present invention contains particles and a solution containing at least the antibody 412. Alternatively, the kit of the present invention may be a kit containing particles on which the antibody 412 is immobilized.

The kit of the present invention is preferably a kit intended for immuno-chromatography. That is, the kit contains an immuno-chromatographic test device on which the antibody 412 is immobilized or carried.

In this specification, the phrase "the antibody is carried" means that the antibody is held so as to be movable.

(Immuno-chromatographic test device)

Accordingly, the present invention also provides an immuno-chromatographic test device on which the antibody 412 is immobilized or carried.

The immuno-chromatographic test device preferably has a sample addition member to which a sample likely of having RSV is added, a label holding member carrying an anti-RSV monoclonal antibody, and a chromatographic membrane carrier (hereinafter referred to as "chromatographic carrier") having a judgment region on which an anti-RSV monoclonal antibody different from the antibody carried on the member holding member is immobilized.

In the immuno-chromatographic test device, the sample addition member and the label holding member are preferably in contact with each other.

The sample addition member and the chromatographic carrier are preferably in contact with each other. The sample addition member is preferably in contact with the chromatographic carrier at the upstream side in the direction of sample development in the chromatographic carrier.

The label holding member is preferably disposed on the upstream side in the direction of sample development in the chromatographic carrier. The label holding member may or may not come into contact with the chromatographic carrier.

In this specification, the terms "upstream side in the direction of sample development" and "downstream side in the direction of sample development" are indicated by relative directions assuming that the side on which a sample is added is the upstream side, while the side toward which a sample is developed (flows) is the downstream side.

(Antibodies for the test device)

In the immuno-chromatographic test device described above, the antibody carried on the label holding member is preferably labeled with the labeling substance described above. The labeling substance is preferably an insoluble granular substance to enable rapid and easy observation of color change with the naked eye, and is particularly preferably colored synthetic polymer particles or metal colloid particles.

In the immuno-chromatographic test device, the antibody labeled with a labeling substance is preferably an anti-RSV monoclonal antibody not binding to a protein having the amino acid sequence of SEQ ID NO: 2, more preferably the antibody 1565.

The anti-RSV monoclonal antibody immobilized on the judgment region is preferably at least one selected from the antibodies 412 and 255, more preferably these two antibodies.

(Sample addition member)

The sample addition member is made preferably of a material such as cotton, glass fibers, a porous synthetic resin such as porous polyethylene or porous polypropylene, or cellulose fibers. The sample addition member can be a product of such a material, such as woven fabric, a nonwoven fabric, a filter paper, a sheet or a film.

(Label holding member)

The label holding member is made preferably of a material such as glass fibers, cellulose fibers, or plastic (for example, polyester, polypropylene or polyethylene) fibers.

Preferably, the label holding member further holds a control labeling substance to confirm whether the sample has been suitably developed or not. The control labeling substance is preferably substantially not reactive to components in the sample. The control labeling substance can be used in combination with the following substance capable of binding to the control labeling substance. That is, the control labeling substance, and the substance capable of binding to the control labeling substance are a combination of two substances capable of binding specifically to each other. Either of the two substances may be used as the control labeling substance. The combination of two substances capable of binding specifically to each other includes a combination of a hapten and a protein capable of binding thereto, for example a combination of biotin and streptavidin or avidin and a combination of 2,4-dinitrophenol or digoxin and a protein (for example, albumin, casein, fibrinogen or the like). Such a combination of a control labeling substance and a substance capable of binding to the control labeling substance is preferably a combination of substances that are not present in the sample.

A label for the control labeling substance may be the same label as the above labeling substance. The control labeling substance may be labeled with the same labeling component as in the antibody or may be labeled with a different labeling component.

(Chromatographic membrane carrier)

The chromatographic carrier is made preferably of a material capable of binding to a protein via physical action such as electrostatic action or hydrophobic interaction and simultaneously developing the sample by capillary phenomenon. Such material of the chromatographic carrier includes nitrocellulose, nylon (for example, a modified nylon into which a carboxyl group, and an amino group which may be substituted with an alkyl group, have been introduced), polyvinylidene difluoride (PVDF), and cellulose acetates.

The chromatographic carrier preferably has a control part on which a substance capable of binding to the control labeling substance is immobilized in order to confirm whether the sample has been suitably developed or not.

The substance capable of binding to the control labeling substance is as described above in connection with the control labeling substance.

(Other members)

The immuno-chromatographic test device can further include an absorption member. The absorption member may use a woven or nonwoven fabric made of a material such as cellulose or glass fibers, or porous plastics such as polyethylene or polypropylene. The absorption member is preferably disposed so as to contact with the chromatographic carrier, at a position downstream, in the direction of sample development, of the chromatographic carrier. By arranging the absorption member at such a position, the test device can increase the development speed of a sample.

The immuno-chromatographic test device preferably has a substrate so that the sample addition member, the label holding member, the chromatographic carrier, and arbitrarily an absorption member can be held together. The substrate is not particularly limited as long as it is used as a substrate for a usual immuno-chromatographic test device. For example, plastics, paper or glass may be used as the substrate. The substrate preferably has a sticky surface to dispose the respective members thereon.

(Method for manufacturing the test device)

The immuno-chromatographic test device of the present invention can be produced in the same manner as for known immuno-chromatographic test devices. That is, the label holding member can be prepared by dipping a member for holding a label, in a suitable buffer solution containing an antibody, preferably a labeled antibody, and then drying it. Alternatively, the label holding member can be prepared by adding the buffer solution to a member for holding a label, and then drying it. The chromatographic carrier can be prepared by allowing a suitable antibody to be held on a judgment region and drying it, thereby immobilizing the antibody on the judgment region. The label holding member, the chromatographic carrier and the sample addition member can be suitably assembled and cut to give the immuno-chromatographic test device.

(Preferable embodiment of the test device)

FIG. 1 shows a sectional view of the immuno-chromatographic test device in one embodiment of the present invention. The immuno-chromatographic test device 1 includes a sample addition member 3 consisting of a nonwoven fabric of cotton, a label holding member 4 consisting of a nonwoven fabric of glass fibers, a chromatographic carrier 5 consisting of a porous body of nitrocellulose, and an absorption member 6 consisting of a nonwoven fabric of cellulose, on a substrate 2 made of a plastic plate having a pressure-sensitive adhesive layer thereon.

The substrate 2 is used in appropriately disposing the members such as the sample addition member 3 and the label holding member 4 thereon. The substrate 2 may be a material such as paper or glass.

A sample is added to the sample addition member 3. The sample is not particularly limited as long as there is a possibility that the sample may contain RSV. The sample is particularly preferably a nasal discharge, a nasal swab or a throat swab. The sample may be added after dilution with a suitable solvent such as a buffer solution.

In this embodiment, the label holding member 4 is disposed in contact with the sample addition member 3 and carries a first antibody (antibody 1565) labeled with a labeling substance.

In this embodiment, the chromatographic carrier 5 is disposed so as not to directly contact the label holding member 4. The chromatographic carrier 5 has a judgment region 5A on which a second antibody reacting, through an antigen-antibody reaction, with a measurement object is immobilized. The second antibody is an antibody different from the first antibody. In this embodiment, both antibodies 412 and 255 are used as the second antibody. The antibody 412 when contained in either the label holding member or the judgment region can be used in combination with another anti-RSV monoclonal antibody besides the anti-RSV monoclonal antibody of the present invention.

The absorption member 6 is disposed in contact with the chromatographic carrier 5 and arranged to absorb an excess of the sample. The absorption member 6 is one capable of rapidly absorbing and maintaining a liquid. A part of the sample addition member 3 and the surface of the absorption member 6 may be covered with a transparent sheet 7 as shown in FIG. 1.

The immuno-chromatographic test device 1 of the present invention can be prepared for example in the following manner.

(1) The chromatographic carrier 5 is placed in the middle of the substrate 2.
(2) At the side of the sample development starting point (that is, the left side of FIG. 1, referred to hereinafter as "upstream side") of the chromatographic carrier 5, the label holding member 4 while being provided with a space so as not to contact the end of the carrier 5 is placed on the upstream side of the substrate 2.
(3) The part, on the upstream side, of the sample addition member 3 is placed on the most upstream side of the substrate 2.
(4) The side of the sample development endpoint (that is, the right side of FIG. 1, referred to hereinafter as "downstream side") of the sample addition member 3 is placed on the upper surface of the label holding member 4 and on the upper surface, on the upstream side, of the chromatographic carrier 5.
(5) The sample addition member 3 is attached to the substrate 2 in the region between the label holding member 4 and the chromatographic carrier 5.
(6) The part, on the upstream side, of the absorption member 6 is placed on the upper surface, on the downstream side, of the chromatographic carrier 5.
(7) The part, on the downstream side, of the absorption member 6 is attached to the most downstream side of the substrate 2.
(8) The part, on the downstream side, of the sample addition member 3 and the surface of the adsorption member 6 are covered with transparent sheets 7 respectively.

The sample is mixed if necessary with a suitable solvent to form a mixed solution capable of chromatographic development. Then, the upstream (sample addition member 3) side of the immuno-chromatographic test device 1 is immersed in the mixed solution, or the mixed solution is added to the sample addition member. By so doing, the mixed solution passes through the sample addition member 3 and mixed with the labeled first antibody in the label holding member 4. If an RSV antigen is present in the mixed solution, the RSV antigen is bound, via an antigen-antibody reaction, to the first antibody to form a complex. This complex moves in the chromatographic carrier 5 and reaches the judgment region 5A. The complex reacts, via an antigen-antibody reaction, with the second antibody immobilized on the judgment region 5A, so that the complex is captured by the judgment region 5A. When colored synthetic polymer particles such as blue latex particles are used as the labeling substance, these particles accumulate thereby staining the judgment region 5A blue. The presence of RSV can thereby be immediately confirmed visually.

The chromatographic carrier 5 may be provided not only with only one judgment region but also with two or more judgment regions. The chromatographic carrier 5 may also be provided with a control portion. When the control portion is arranged, for example, a labeling substance (for example red latex particles)-labeled avidin is movably held on the label holding member 4, and biotin capable of specific binding to avidin is immobilized on the control portion in the chromatographic carrier 5.

The kit for detection of RSV and the immuno-chromatographic test device in the present invention, as compared with conventional RSV detection methods, can improve the sensitivity of RSV detection. Accordingly, the present invention enables more accurate detection of RSV to be effected rapidly and easily, thus providing an indicator for more accurate diagnosis of RSV infection.

Examples

The present invention will be described in more detail with reference to the Examples, but the present invention is not limited to these examples.

Example 1

Preparation of Hybridoma

(Immunization of mouse)

100 $\mu$L of a phosphate buffer solution (PBS) containing a commercial RSV antigen obtained from Capricon was mixed with 100 $\mu$L of Freund's complete adjuvant (FCA) and emulsified to prepare 200 $\mu$L of an FCA RSV antigen solution. Separately, 200 $\mu$L of an FIA RSV antigen solution was prepared in the same manner as described above except that Freund's incomplete adjuvant (FIA) was used in place of FCA.

A 7- to 8-week-old female Balb/c mouse was initially immunized by intraperitoneal injection of 200 $\mu$L of the FCA RSV antigen solution. After the initial immunization, booster immunization with 200 $\mu$L of the FIA RSV antigen solution was carried out every 2 to 3 weeks. Ten days and three days before removal of splenocytes, 200 $\mu$L of an RSV antigen (manufactured by Capricon) was intravenously injected. Three days after final administration, splenocytes were separated and fused with mouse myeloma cells P3X63-Ag8.653 to prepare hybridomas.

(Culture of the Hybridomas)

The hybridomas were suspended at a density of $2.5 \times 10^6$ cells/mL in HAT medium and dispensed to each well ($2.5 \times 10^5$ cells/well) of a 96-well plate (manufactured by Corning Inc.; referred to hereinafter as culture plate). The culture plate was left in an 8% $CO_2$ incubator at 37°C, to initiate culture of the hybridomas. When the cells were cultured for 10 or more days until hybridoma colonies appeared, a monoclonal antibody-producing hybridoma was screened.

(Screening of the Hybridomas)

An RSV antigen (manufactured by Capricon) was added to a concentration of 0.5 $\mu$g/mL to 0.1 M phosphate buffer solution (PBS) (pH 7.5) containing 0.1 w/v% $NaN_3$, to prepare an RSV antigen solution for immunization. 100 $\mu$L of this RSV antigen solution for immunization was dispensed into each well of an immunomodule (manufactured by NUNC) (hereinafter, the immunomodule will be referred to as antigen-immobilized plate). The antigen-immobilized plate was left overnight at 4°C and then washed 3 times with a PBS buffer solution containing Tween 20 at a concentration of 0.05% (hereinafter, referred to as buffer solution A). After washing, 300 $\mu$L of PBS containing BSA at a concentration of 1 w/v% (hereinafter, referred to as buffer solution B) was added to each well of the antigen-immobilized plate and left for at least 4 hours at 2°C to 8°C. The antigen-immobilized plate was stored at 2°C to 8°C before use.

The buffer solution B was removed from the antigen-immobilized plate.
After removal of the buffer solution, 75 $\mu$L of buffer solution B was added to each well of the antigen-immobilized plate.

A culture supernatant of the hybridoma described above was removed from each well of the culture plate and then added in a volume of 25 μL to each well of the antigen-immobilized plate. After the buffer solution B and the culture supernatant were added, the plate was stirred at room temperature for 1 hour. After stirring, each well of the antigen-immobilized plate was washed 3 times with buffer solution A. After washing, 100 μL of horseradish peroxidase (POD)-labeled anti-mouse Ig polyclonal antibody (Code No. P0447, manufactured by DAKO) was added to each well of the antigen-immobilized plate and incubated at room temperature for 30 minutes. After incubation, each well of the antigen-immobilized plate was washed 3 times with buffer solution A. After washing, 100 μL of a substrate solution containing ortho-phenylene diamine (OPD) as a substrate of POD was added to each well and then left at room temperature for 10 minutes. Then, 100 μL of a reaction termination solution containing 2 N $H_2SO_4$ was added to each well of the antigen-immobilized plate. The reaction solution in each well was measured for its absorbance at 492 nm with a microplate reader (manufactured by Molecular Devices).

The resulting three hybridomas RSF1-1565, RSF2-412 and RSF6-255 have been internationally deposited (Accession No. FERM BP-11119, Accession No. NITEBP-601, and Accession No. NITE BP-602, respectively).

Example 2

Confirmation of the reactivity of anti-RSV-F-protein monoclonal antibodies

(Confirmation of the reactivity of antibodies 1565, 412 and 255)

As RSV-F antigens, Long strain (10 [6.7] TCID (50)/ml), A2 strain (10 [5.5] TCID (50)/ml), 9320 strain (10 [5.5] TCID (50)/ml) and B1 wild-type (BWV) strain (10 [4.5] TCID (50)/ml) were used. These 4 strains of RSV can be purchased from American Type Culture Collection (ATCC). Each of the RSV strains was diluted to 1/10 with a phosphate buffer solution containing bovine serum albumin (BSA) (hereinafter referred to as first buffer solution).

Each diluted antigen solution and an analyte extracting reagent (100 mM citric acid/0.4 M NaCl, 10 mM dithiothreitol, 0.1 (v/v) % polyoxyethylene(9) octylphenyl ether) were mixed with each other in equal volumes and then subjected to extraction treatment for 5 minutes to prepare each antigen extract.

A Sepharose bead solution in which Sepharose beads (manufactured by Amersham Biosciences) to which commercial anti-mouse antibody IgG had been bound were contained at a concentration of 15 v/v % was prepared.

The Sepharose bead solution, each antigen extract described above, and the first buffer solution were mixed to prepare each antigen sample. The amounts of the Sepharose bead solution and each antigen extract in each antigen sample are shown in Table 1.

Table 1

|  | Sepharose bead solution | Antigen extract | First buffer solution |
|---|---|---|---|
| Long strain antigen sample | 1 ml | 0.2 ml | 0.8 ml |
| A2 strain antigen sample | 1 ml | 0.1 ml | 0.9 ml |
| 9320 strain antigen sample | 1 ml | 0.05 ml | 0.95 ml |
| BWV strain antigen sample | 1 ml | 0.2 ml | 0.8 ml |

The antigen sample was added in a volume of 60 μL/well to each well of a V-shaped 96-well plate (manufactured by Sanplatec Co., Ltd.). Then, the antibody 1565, antibody 412 and antibody 255 were diluted to 1 μg/ml with the first buffer solution to prepare an antibody 1565 solution, an antibody 412 solution and an antibody 255 solution, respectively. The prepared antibody solutions were added separately to wells in a volume of 30 μL/well. Thereafter, the V-shaped 96-well plate was stirred at room temperature for 60 minutes.
After stirring, the V-shaped 96-well plate was left for 10 minutes.

A solution of antibody 133-1 H (antibody against RSV; manufactured by CHEMICON) diluted to a concentration of 10 μg/mL with 0.1 M sodium phosphate buffer solution was dispensed in a volume of 100 μL to each well of an immunomodule (manufactured by NUNC). Each well was washed 3 times with a Tween 20-containing phosphate buffer solution (referred to hereinafter as second buffer solution). After washing, each well was blocked by dispensing 300 μL of the first buffer solution to each well (hereinafter, this immunomodule will be referred to as antibody 133-1 H-sensitized plate),

The first buffer solution was removed from the antibody 133-1 H-sensitized plate. After removal of the buffer solution, 50 μL of the supernatant in each well of the above V-shaped 96-well plate which had been left for 10 minutes was added to each well of the antibody 133-1 H-sensitized plate. Then, the antibody 133-1 H-sensitized plate was stirred at room temperature for 30 minutes, and then the antibody 133-1 H-sensitized plate was washed 3 times with the second buffer solution.

100 $\mu$L of a first buffer solution containing biotin-labeled antibody B016 (final concentration of 2 $\mu$g/mL, manufactured by Serotec) and streptavidin-labeled peroxidase (POD, final concentration of 50 mU/mL) was added to each well of the antibody 133-1 H-sensitized plate after washing. Then, the antibody 133-1 H-sensitized plate was stirred at room temperature for 60 minutes. Thereafter, the antibody 133-1 H-sensitized plate was washed 3 times with the second buffer solution.

100 $\mu$L of a substrate solution containing ortho-phenylene diamine (OPD) as a substrate of POD was added to each well of the antibody 133-1 H-sensitized plate and then left at room temperature for 10 minutes. Then, 100 $\mu$L of a reaction termination solution containing 2 N $H_2SO_4$ was added to each well of the antibody 133-1 H-sensitized plate, and then the reaction solution in each well was measured for its absorbance (absorbance A) at 492 nm with a microplate reader (manufactured by Molecular Devices).

In a control experiment, a control sample in which the antibody solution had been changed to the first buffer solution was prepared and examined in the same experiment as described above to determine the absorbance (absorbance B). In another control experiment, a control sample in which the antibody solution and the antigen sample had been changed to the first buffer solution was prepared and examined in the same experiment as described above to determine the absorbance (absorbance C).

Using the absorbance A to C thus obtained, the degree of absorption of each measurement sample by each hybridoma-derived anti-RSV-F-protein monoclonal antibody was determined according to the following equation (1):

$$\text{Degree of absorption (\%)} = [(1-(A-C)/(B-C)]\times100 \qquad (1)$$

The evaluation results of the reactivity of each anti-RSV-F-protein monoclonal antibody are shown in Table 2. In Table 2, "+++°°" shows an absorption of 90% or more as determined by the equation (1); "++", 50% or more; "+", 30% or more; and "-", less than 30%.

Table 2

| Monoclonal antibody | Used RSV strains | | | |
|---|---|---|---|---|
| Antibody clone name | Long strain | A2 strain | 9320 strain | B1 wild-type strain |
| RSFI-1565 | +++ | +++ | +++ | +++ |
| RSF2-412 | ++ | ++ | + | ++ |
| RSF6-255 | +++ | +++ | +++ | +++ |

As being clear from the results in Table 2, it has revealed that the antibodies 1565, 412 and 255 exhibit reactivity to any antigens of Long strain, A2 strain, 9320 strain and BWV strain.

Example 3

Confirmation of antigen recognition sites of the anti-RSV-F-protein monoclonal antibodies

(Test of inhibition on antibody B016)

The A2 strain (10 [5.5] TCID (50)/ml) was diluted to 1/10 with the first buffer solution. The diluted antigen solution and an analyte extracting reagent were mixed with each other in equal volumes and then subjected to extraction treatment for 5 minutes to prepare an A2 strain antigen extract. Then, the A2 strain antigen extract was diluted to 1/20 with the first buffer solution to prepare an antigen sample.

A solution of antibody 133-1 H (manufactured by CHEMICON) diluted to a concentration of 10 $\mu$g/mL with 0.1 M sodium phosphate buffer solution was dispensed in a volume of 100 $\mu$L to each well of an immunomodule (manufactured by NUNC). Each well was washed 3 times with the second buffer solution. After washing, each well was blocked by dispensing 300 $\mu$L of the first buffer solution to each well (hereinafter, this immunomodule will be referred to as antibody 133-1 H-sensitized plate).

The first buffer solution was removed from the antibody 133-1 H-sensitized plate. After removal of the buffer solution, 100 $\mu$L of the antigen sample was added to each well of the antibody 133-1 H-sensitized plate which was then stirred at room temperature for 30 minutes. Thereafter, each well of the antibody-sensitized plate was washed 3 times with the second buffer solution.

The antibodies 1565, 412 and 255 were diluted to 10 $\mu$g/mL with the first buffer solution respectively, to prepare an antibody 1565 solution, an antibody 412 solution, and an antibody 255 solution. The prepared antibody solutions were added separately in a volume of 50 $\mu$L/well to each well of the antibody 133-1 H-sensitized plate. Then, 50 $\mu$L first buffer solution containing biotin-labeled antibody B016 (antibody against RSV; final concentration of 2 $\mu$g/ml; manufactured by UK-Serotec) and streptavidin-labeled POD (final concentration 40 mU/ml) was added to each well of the antibody 133-1 H-sensitized plate, and then the antibody 133-1 H-sensitized plate was stirred at room temperature for 60 minutes and then the antibody 133-1 H-sensitized plate was washed 3 times with the second buffer solution.

100 $\mu$L of a substrate solution containing OPD was added to each well of the antibody 133-1 H-sensitized plate and then left at room temperature for 10 minutes. Then, 100 $\mu$L of a reaction termination solution containing 2 N $H_2SO_4$ was added to each well of the antibody 133-1 H-sensitized plate, and the reaction solution in each well was measured for its absorbance at 492 nm with a microplate reader (absorbance A).

In a control experiment, a control sample in which the antibody solution had been changed to the first buffer solution was prepared and examined in the same experiment as described above to determine the absorbance (absorbance B). In another control experiment, a control sample in which the antibody solution and the antigen sample had been changed to the first buffer solution was prepared and examined in the same experiment as described above to determine the absorbance (absorbance C).

Using the absorbance A to C thus obtained, the degree of inhibition of each measurement sample by each hybridoma-derived anti-RSV-F-protein monoclonal antibody was determined according to the following equation (2):

$$\text{Inhibition (\%)} = [(1-(A-C)/(B-C)] \times 100 \quad (2)$$

(Test of inhibition on antibody 133-1 H)

An experiment was conducted in the same manner as in the above test of inhibition on the antibody B016 except that an antibody B016-sensitized plate having an immunomodule sensitized in each well with the antibody B016 was used in place of the antibody 133-1 H-sensitized plate, and a biotin-labeled antibody 133-1 H was used in place of the biotin-labeled antibody B016.

The evaluation results of the inhibition of each anti-RSV-F-protein monoclonal antibody on the antibodies 133-1 H and B016 are shown in Table 3. In Table 3, "+++" shows an inhibition degree of 90% or more as determined by the equation (2); "++", 50% or more; "+", 30% or more; and "-", less than 30%.

Table 3

| Antibody clone name | Inhibition on antibody 133-1H | Inhibition on antibody B016 |
|---|---|---|
| RSF1-1565 | +++ | - |
| RSF2-412 | - | - |
| RSF6-255 | - | +++ |

From the results in Table 3, it has revealed that the antibody 412 has an antigen recognition site different from the antigen recognition sites of the antibodies 133-1 H and B016.

Example 4

Test of detection of RSV antigen by a combination of RSV-F-protein monoclonal antibodies

The Long strain (10 [6.7] TCID (50)/ml) was diluted to 1/10 with the first buffer solution. The diluted antigen solution and an analyte extracting reagent were mixed with each other in equal volumes and then subjected to extraction treatment for 5 minutes to prepare a Long strain antigen extract.

The antibodies 1565, 412 and 255 diluted to a concentration of 5 $\mu$g/mL with 0.1 M sodium phosphate buffer solution were dispensed in a volume of 100 $\mu$L/well to each well of immunomodules, respectively. Each well was washed 3 times with the second buffer solution. After washing, each well was blocked by dispensing 300 $\mu$L of the first buffer solution to each well, to prepare an antibody 1565-sensitized plate, an antibody 412-sensitized plate and an antibody 255-sensitized plate.

The first buffer solution was removed from each of the prepared antibody-sensitized plates. After removal of the buffer solution, 50 μL of the Long strain antigen extract was added to each well of each antibody-sensitized plate. Each antibody-sensitized plate was stirred at room temperature for 30 minutes and then washed 3 times with the second buffer solution.

The antibodies 1565, 412 and 255 were labeled with biotin by a known method, thereby preparing a biotin-labeled antibody RSF1-1565, a biotin-labeled antibody RSF2-412 and a biotin-labeled antibody RSF6-255.

A first buffer solution containing each of the prepared biotin-labeled antibodies (final concentration 2 μg/ml) and streptavidin-labeled POD (final concentration 40 mU/ml) was added in a volume of 100 μL to each of the antibody-sensitized plates. Each of the antibody-sensitized plates was stirred at room temperature for 60 minutes and then washed 3 times with the second buffer solution.

100 μL of a substrate solution containing OPD was added to each well of each antibody-sensitized plate and then left at room temperature for 10 minutes. Then, 100 μL of a reaction termination solution containing 2 N $H_2SO_4$ was added to each well of the antibody-sensitized plates. The reaction solution in each well was measured for its absorbance at 492 nm with a microplate reader. The measurement results are shown in Table 4.

Table 4

|  |  | On the side of the solid phase | | |
|---|---|---|---|---|
|  |  | RSF1-1565 | RSF2-412 | RSF6-255 |
| On the side of the label | RSF1-1565 | 0.049 | 1.579 | 1.551 |
|  | RSF2-412 | 2.250 | 0.011 | 1.812 |
|  | RSF6-255 | 2.383 | 1.499 | 0.006 |

From the results in Table 4, it has revealed that the antibodies 1565, 412 and 255, unless the same antibody is used as antibodies on the solid phase side and on the label side, can be combined with each other to detect the RSV antigen by sandwich immunoassay.

Example 5

Analysis of an epitope of the antibody 1565

(Preparation of an antibody-1565 escape mutant)

Hep2 cells for infection with RSV were cultured on a 24-well plate (manufactured by Corning) with Eagle MEM (manufactured by SIGMA) containing 10% fetal bovine serum. When the Hep2 cells became confluent, the culture medium was exchanged with Eagle MEM containing 1% fetal bovine serum, and the cells were infected at an MOI of 0.0005 with the Long strain (10 [6.7] TCID (50)/ml). After infection, the cells were incubated for 1 hour at 37°C in 5% $CO_2$. After incubation for 1 hour, the antibody 1565 was added in a volume of 20 μg/well to the cells. After the antibody was added, the cells were cultured at 37°C in 5% $CO_2$ for 3 to 4 days.

A culture medium was removed from HeP2 cells cultured separately in Eagle MEM containing 10% fetal bovine serum, and to the HeP2 cells was added half of a culture supernatant of the above cells which had been cultured for 3 to 4 days. Further, Eagle MEM containing 1% fetal bovine serum in an amount equal to the culture supernatant of the cells was added thereto. After addition, the cells were incubated for 1 hour at 37°C in 5% $CO_2$. After incubation for 1 hour, the antibody 1565 was added in a volume of 20 μg/well. After the antibody was added, the cells were cultured at 37°C in 5% $CO_2$ for 3 to 4 days. This procedure was carried out further 5 times.

After the cells infected with RSV were subcultured 7 times in total in the presence of the antibody 1565, cytopathic effect was observed. The appearance of an antibody-1565 escape mutant could be confirmed by observing the cytopathic effect, so the whole culture of the cells was recovered and stored in a frozen state.

(Gene sequence analysis and amino acid sequence analysis of RSV-F protein of the antibody-1565 escape mutant)

From a culture supernatant of the antibody-1565 escape mutant and the Long strain, cDNA was synthesized by using ReverTra Ace-α (Product Code FSK-101, manufactured by Toyobo) and a primer [sequence: ATGGAGTTGCCAATCCTCAAAGC (SEQ ID NO: 3)].

Each synthesized cDNA was subjected to polymerase chain reaction (PCR) with PrimeStar (Product Code R010A, manufactured by TAKARA) and primers [sequences: ATGGATCCATGGAGTTGCCAATCCTCAAAGC (SEQ ID NO: 4) and

TAGAATTCTAGTGATGGTGATGGTGATGATTTGTGGTGGATTTACCAGCATT (SEQ ID NO: 5)]. PCR involved a reaction at 95°C for 2 minutes followed by 35 cycles each consisting of a reaction at 95°C for 30 seconds, at 55°C for 20 seconds and at 72°C for 100 seconds. After the reaction was finished, the PCR product was stored in a frozen state.

Using MinElute PCR Purification Kit (QIAGEN), the primers were removed from the PCR product. The PCR product from which the primers had been removed was subjected to cycle sequencing reaction with BigDye Terminator v3.1 Cycle Sequencing Kit (manufactured by Applied Biosystems) and primers [sequences: ATGGATCCATGGAGTTGCCAATCCTCAAAGC (SEQ ID NO: 4), TTTCTTGGTTTTTTGTTAGGTGTTGG (SEQ ID NO: 6), TTTAACATTACCAAGTGAAATAAATCT (SEQ ID NO: 7), and

TCTTCTTTTCCTTTTCTTGCTTAATG (SEQ ID NO: 8)]. After the reaction, the labeled nucleotide was removed, and gene sequence analysis and predicted amino acid sequence analysis were performed with a sequencer (manufactured by Applied Biosystems) and sequence analysis software (manufactured by Hitachi Software).

The amino acid sequence of the RSV-F protein of the antibody-1565 escape mutant is set forth in SEQ ID NO: 2. The amino acid sequence of the RSV-F protein of the Long strain is set forth in SEQ ID NO: 1. As being clear from SEQ ID NOS: 1 and 2, it has revealed that in the RSV-F protein of the antibody-RSF1-1565 escape mutant, lysine (K) at position 421 had been changed to threonine (T).

(Analysis of reactivity with the antibody-1565 escape mutant)

A culture supernatant of the antibody-1565 escape mutant and the Long strain were diluted to 1/10 with the first buffer solution, respectively. Each of the diluted antigen solutions and an analyte extracting reagent were mixed with each other in equal volumes and then subjected to extraction treatment for 5 minutes to prepare each antigen extract. Then, each antigen extract was diluted to 1/5 with the first buffer solution to prepare an antigen sample.

100 $\mu$L of a solution of antibody B016 (manufactured by UK-Serotec) diluted to a concentration of 10 $\mu$g/mL with 0.1 M sodium phosphate buffer solution was dispensed to each well of an immunomodule (manufactured by NUNC). Each well was washed 3 times with a Tween 20-containing phosphate buffer solution (referred to hereinafter as second buffer solution). After washing, each well was blocked by dispensing 300 $\mu$L of the first buffer solution to each well (hereinafter, this immunomodule will be referred to as antibody B016-sensitized plate).

The first buffer solution was removed from the antibody B016-sensitized plate. After removal of the buffer solution, 100 $\mu$L of the antigen sample was added to each well of the antibody B016-sensitized plate and then stirred for 30 minutes. Thereafter, each well of the antibody B016-sensitized plate was washed 3 times with the second buffer solution.

The antibody 1565 was labeled with biotin by a known method, thereby preparing a biotin-labeled antibody RSF1-1565.

A first buffer solution containing the prepared biotin-labeled antibody 1565 (final concentration 2 $\mu$g/ml) and streptavidin-labeled POD (final concentration 40 mU/ml) was added in a volume of 100 $\mu$L to each well of the antibody B016-sensitized plate, and the antibody B016-sensitized plate was stirred at room temperature for 60 minutes. Thereafter, the antibody B016-sensitized plate was washed 3 times with the second buffer solution.

100 $\mu$L of a substrate solution containing OPD was added to each well of the antibody B016-sensitized plate and then left at room temperature for 10 minutes. Then, 100 $\mu$L of a reaction termination solution containing 2 N $H_2SO_4$ was added to each well of the antibody B016-sensitized plate, and then the reaction solution in each well was measured for its absorbance at 492 nm with a microplate reader. The measurement results of the absorbance are shown in Table 5.

Table 5

|  | Absorbance |
| --- | --- |
| Antibody-RSF1-1565 escape mutant | 0.03 |
| Long strain | 3.28 |

From Table 5, it has revealed that the antibody 1565 does not recognize the RSV-F protein of the antibody-1565 escape mutant.

Example 6

Preparation of the immuno-chromatographic test device for detection of RSV

(Preparation of the test device of the present invention)

The antibody 1565 was used as the first antibody labeled with a labeling substance, and the antibodies 412 and 255 were used as the second antibody immobilized on a chromatographic carrier, and an immuno-chromatographic test device 1 (referred to hereinafter as test device) as shown in FIG. 1 was prepared by according to the following method.

First, the antibodies 412 and 255 were diluted to a final concentration of 1.0 mg/mL with a phosphate buffer solution, pH 7.0, to prepare a mixed solution of the antibodies 412 and 255. Then, the mixed solution of the antibodies 412 and 255 was applied in 1-mm width via an antibody applicator (BioDot Ltd.) onto a judgment part 5A of a chromatographic carrier made of a nitrocellulose membrane, and then dried at 50°C for 30 minutes.

After drying, the chromatographic carrier 5 was blocked by dipping in a blocking solution (BSA-containing phosphate buffer solution, pH 7.0). Thereafter, the chromatographic carrier 5 was washed with a washing solution(SDS-containing phosphate buffer solution, pH 7.0) and dried at 40°C for 120 minutes to complete the chromatographic carrier 5.

Then, blue colored polystyrene latex particles (particle size 0.3 $\mu$m) were sensitized with the antibody 1565 and then suspended in a dispersing buffer solution (phosphate buffer solution, pH 7.0, containing BSA and sucrose), to prepare antibody 1565-sensitized latex particles. In this sensitization, the antibody was used at a concentration of 200 $\mu$g IgG per mL of 1% latex particles. The antibody 1565-sensitized latex particles were added to a glass fiber pad and then dried in a vacuum drier to prepare a label holding member 4.

The chromatographic carrier 5 and the label holding member 4 were used to produce the test device of the present invention.

(Preparation of a control test device)

A control test device was prepared in the same manner as in preparation of the test device of the present invention except that the antibody B016 was used as the first antibody, the antibody 133-1 H was used as the second antibody, and an antibody B016 solution diluted to a final concentration of 2.0 mg/mL was used in place of the mixed solution of the antibodies 412 and 255.

Example 7

Confirmation of the reactivity of the test device of the present invention and conventional test devices to RSV

(Detection of RSV by the test device of the present invention)

Using the test device of the present invention prepared in Example 6, its reactivity to RSV was confirmed in the following procedures.

(1) Three types of RSV (Long strain, BWV strain and 9320 strain) shown in Table 6 were cultured in Vero cells, and the resulting viruses were diluted to degrees of dilution shown in Table 6 with physiological saline to prepare viral solutions.
(2) 150 $\mu$L of the viral solution diluted at a predetermined degree of dilution, obtained in (1) above, was added to and mixed with 800 $\mu$L of an analyte extracting reagent (phosphate buffer solution, pH 7.3, containing 0.3 w/v% NP-40 (polyoxyethylene(9) octylphenyl ether), to prepare a measurement sample.
(3) 200 $\mu$L of the measurement sample prepared in (2) above was dropped into a glass test tube, and the upstream (sample addition member 3) side of the test device of the present invention was dipped and left in the measurement sample in the glass test tube, and about 10 minutes thereafter, coloration in the judgment region 5A was judged with the naked eye. Judgment was made in which (+) was given when coloration was recognized, and (-) was given when no coloration was recognized. The results are shown in Table 6.

(Detection of RSV by conventional test devices)

As conventional test devices, the control test device and commercial test devices Check RSV (manufactured by Alfresa-Pharma Co. Japan) and BD RSV Examan (manufactured by Becton Dickinson) were used to confirm their reactivity to RSV.

The reactivity of the control test device to RSV was confirmed in the same manner as described above for the test device of the present invention. The reactivity of the commercial test devices to RSV was confirmed using the above viral solutions, according to the operation described in a material attached to each of the commercial test devices. The results of confirmation of the reactivity are shown in Table 6.

Table 6

| Solid Phase | Degree of Dilution | Control Test Device | Test Device of the Present Invention | Commercial Test Devices | |
|---|---|---|---|---|---|
| Label | | 133-1H / B016 | Antibody 412 / Antibody 255 / Antibody 1565 | Check RSV | RSV Examan |
| Long | 1:2.5 | + | NT | NT | NT |
| | 1:5 | - | NT | NT | + |
| | 1:10 | NT | + | + | - |
| | 1:20 | NT | - | - | - |
| | 1:40 | NT | NT | NT | NT |
| BWV | 1:2.5 | + | NT | NT | + |
| | 1:5 | + | NT | NT | - |
| | 1:10 | - | NT | NT | - |
| | 1:20 | NT | NT | + | - |
| | 1:40 | NT | NT | - | NT |
| | 1:80 | NT | NT | NT | NT |
| | 1:160 | NT | + | NT | NT |
| | 1:320 | NT | - | NT | NT |
| 9320 | 1:2.5 | NT | NT | NT | + |
| | 1:5 | + | NT | NT | - |
| | 1:10 | + | NT | NT | - |
| | 1:20 | NT | NT | + | - |
| | 1:40 | NT | NT | - | NT |
| | 1:80 | NT | NT | - | NT |
| | 1:160 | NT | + | NT | NT |
| | 1:230 | NT | - | NT | NT |

NT: Not tested

As being clear from Table 6, the test device of the present invention showed higher reactivity to RSV than by the conventional test devices.

**Claims**

1. A kit for detection of RS virus (RSV), comprising an RSV F protein-recognizing anti-RSV monoclonal antibody produced by hybridoma RSF2-412 deposited on April 20, 2009, under Accession No. NITE BP-601, with Incorporated Administrative Agency National Institute of Technology and Evaluation Patent Microorganisms Depositary.

2. The kit according to claim 1, which further comprises at least one RSV F protein-recognizing anti-RSV monoclonal antibody which is an antibody produced by hybridoma RSF1-1565 deposited on April 20, 2009, under Accession No. FERM BP-11119, with International Patent Organism Depositary National Institute of Advanced Industrial Science and Technology or by hybridoma RSF6-255 deposited on April 20, 2009, under Accession No. NITE BP-602, with Incorporated Administrative Agency National Institute of Technology and Evaluation Patent Microorganisms Depositary.

3. The kit according to claim 1 or 2, wherein at least one of the anti-RSV monoclonal antibodies used in the kit is labeled with a labeling substance.

4. The kit according to claim 3, wherein the anti-RSV monoclonal antibody labeled with a labeling substance is an anti-RSV monoclonal antibody produced by the hybridoma RSF1-1565.

5. The kit according to claim 1, 2, 3 or 4, comprising at least one anti-RSV monoclonal antibody immobilized on a solid phase.

6. The kit according to claim 5, wherein the at least one anti-RSV monoclonal antibody immobilized on a solid phase is selected from the anti-RSV monoclonal antibodies produced by the hybridoma RSF2-412 and the antibodies produced by the hybridoma RSF6-255.

7. An immuno-chromatographic test device for detection of RSV, comprising at least an RSV F protein-recognizing anti-RSV monoclonal antibody produced by hybridoma RSF2-412.

8. The immuno-chromatographic test device according to claim 7, which further comprises at least one of RSV F protein-recognizing anti-RSV monoclonal antibody selected from an antibody produced by hybridoma RSF1-1565 and an antibody produced by hybridoma RSF6-255.

9. The immuno-chromatographic test device according to claim 7 or 8, comprising:

   a sample addition member to which a sample likely of containing RSV is added;
   a label holding member on which an anti-RSV monoclonal antibody is carried; and
   a chromatographic carrier having a judgment region on which at least one anti-RSV monoclonal antibody different from the antibody carried on the label holding member is immobilized;

   wherein the antibody carried on the label holding member is labeled with a labeling substance.

10. The immuno-chromatographic test device according to claim 9, wherein the antibody labeled with a labeling substance is an anti-RSV monoclonal antibody produced by hybridoma RSF1-1565.

11. The immuno-chromatographic test device according to claim 9 or 10, wherein the labeling substance is an insoluble granular substance.

12. The immuno-chromatographic test device according to claim 11, wherein the insoluble granular substance is selected from the group consisting of colored synthetic polymer particles and metal colloid particles.

13. The immuno-chromatographic test device according to claim 9, 10, 11 or 12, wherein the at least one anti-RSV monoclonal antibody immobilized on the judgment region is an antibody selected from the group consisting of an anti-RSV monoclonal antibody produced by hybridoma RSF2-412 and an anti-RSV monoclonal antibody produced by hybridoma RSF6-255.

14. An anti-RSV monoclonal antibody recognizing an RS virus F protein, which is selected from the group consisting of an antibody produced by hybridoma RSF2-412, an antibody produced by hybridoma RSF1-1565, and an antibody produced by hybridoma RSF6-255.

FIG. 1

Europäisches
Patentamt

European
Patent Office

Office européen
des brevets

## EUROPEAN SEARCH REPORT

**Application Number**

EP 09 16 5635

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SLINGER ROBERT ET AL: "Evaluation of the QuickLab RSV test, a new rapid lateral-flow immunoassay for detection of respiratory syncytial virus antigen." JOURNAL OF CLINICAL MICROBIOLOGY AUG 2004, vol. 42, no. 8, August 2004 (2004-08), pages 3731-3733, XP002546680 ISSN: 0095-1137 * the whole document * | 1-14 | INV. C07K16/10 G01N33/569 |
| X | BECTON, DICKINSON AND COMPANY: "Understanding RSV Testing" TECHNICAL BULLETIN, [Online] vol. 1, no. 1, September 2004 (2004-09), pages 1-8, XP002546681 Retrieved from the Internet: URL:http://www.bd.com/ds/technicalCenter/t echnicalBulletins/tb_0_2689.pdf> [retrieved on 2009-09-21] * the whole document * | 1-14 | |
| D,X | US 5 866 125 A (BRAMS PETER [US] ET AL) 2 February 1999 (1999-02-02) * examples 1-6 * | 1-14 | TECHNICAL FIELDS SEARCHED (IPC) C07K G01N |
| X | US 2006/159695 A1 (DELVECCHIO ALFRED [US] ET AL) 20 July 2006 (2006-07-20) * examples 1,10 * | 1-14 | |
| D,X | WO 03/063767 A (CENTOCOR [US]) 7 August 2003 (2003-08-07) * example 1 * * claims 1-7 * | 1-14 | |

The present search report has been drawn up for all claims

| Place of search | Date of completion of the search | Examiner |
|---|---|---|
| Munich | 21 September 2009 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 09 16 5635

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2009

| Patent document cited in search report | | Publication date | Patent family member(s) | | Publication date |
|---|---|---|---|---|---|
| US 5866125 | A | 02-02-1999 | AT | 431159 T | 15-05-2009 |
| | | | AU | 717061 B2 | 16-03-2000 |
| | | | AU | 6172096 A | 30-12-1996 |
| | | | BR | 9608884 A | 06-07-1999 |
| | | | CA | 2223033 A1 | 19-12-1996 |
| | | | CN | 1192695 A | 09-09-1998 |
| | | | CN | 1680447 A | 12-10-2005 |
| | | | CO | 4480110 A1 | 09-07-1997 |
| | | | EP | 2095827 A1 | 02-09-2009 |
| | | | EP | 0854730 A1 | 29-07-1998 |
| | | | HK | 1015282 A1 | 02-12-2005 |
| | | | JP | 3771267 B2 | 26-04-2006 |
| | | | JP | 11507640 T | 06-07-1999 |
| | | | MX | PA01011457 A | 08-09-2005 |
| | | | NO | 975621 A | 04-02-1998 |
| | | | NZ | 310847 A | 28-05-1999 |
| | | | NZ | 335241 A | 29-07-1999 |
| | | | WO | 9640252 A1 | 19-12-1996 |
| | | | US | 5811524 A | 22-09-1998 |
| | | | US | 5840298 A | 24-11-1998 |
| | | | US | 5939068 A | 17-08-1999 |
| | | | US | 5955364 A | 21-09-1999 |
| | | | US | 5958765 A | 28-09-1999 |
| | | | ZA | 9604434 A | 10-12-1996 |
| US 2006159695 | A1 | 20-07-2006 | AR | 051942 A1 | 21-02-2007 |
| WO 03063767 | A | 07-08-2003 | CA | 2466025 A1 | 07-08-2003 |
| | | | EP | 1501870 A2 | 02-02-2005 |
| | | | JP | 2005522996 T | 04-08-2005 |
| | | | MX | PA04004224 A | 31-03-2005 |

**EP 2 145 899 A1**

REFERENCES CITED IN THE DESCRIPTION

**Patent documents cited in the description**

- WO 9819704 A
- WO 03063767 A
- WO 9640252 A